# EUROPEAN PATENT SPECIFICATION

(11) **EP 1 733 723 B1**
(45) Date of publication and mention of the grant of the patent: **13.07.2011**
(21) Application number: 05710637.9
(22) Date of filing: 24.02.2005
(51) Int. Cl.: A61K 31/202, A61K 45/00, A61P 9/00, A61P 9/10, A61P 43/00

(54) **MEDICINE CAPABLE OF INHIBITING ACTIVATION OF TRANSCRIPTION FACTOR KLF5**
MEDIKAMENT ZUR HEMMUNG DER AKTIVIERUNG DES TRANSKRIPTIONSFAKTORS KLF5
MEDICAMENT CAPABLE D'INHIBER L'ACTIVATION DU FACTEUR DE TRANSCRIPTION KLF5

(30) Priority: 25.02.2004 JP 2004049282
(43) Date of publication of application: 20.12.2006
(73) Proprietor: The University of Tokyo, Bunkyo-Ku, Tokyo 113-0033 (JP); Kowa Company, Ltd., Nagoya-shi, Aichi 460-8625 (JP)
(72) Inventor: NAGAI, Ryozo, 1130033 (JP); SUZUKI, Toru, 1500022 (JP)
(74) Representative: Polypatent
(86) International application number: PCT/JP2005/002986
(87) International publication number: WO 2005/079783

(56) References cited:
- JP-A- 4 178 348
- JP-A- 7 112 930
- JP-A- 10 316 617
- JP-A- 56 140 949
- JP-A- 59 101 448
- JP-A- 61 210 050
- SHINDO T ET AL: "Kru"ppel-like zinc-finger transcription factor KLF5/BTEB2 is a target for angiotensin II signaling and an essential regulator of cardiovascular remodeling" NATURE MEDICINE 2002 US, vol. 8, no. 8, 2002, pages 856-863, XP002539843 ISSN: 1078-8956
- KOWASE KEIKO KAWAI ET AL: "Retinoic acid inhibits BTEB2 gene expression and its function in vascular smooth muscle cells: The potential role of BTEB2 in retinoic acid-mediated inhibition of neointimal formation after balloon injury" CIRCULATION, LIPPINCOTT WILLIAMS & WILKINS, US, vol. 102, no. 18, suppl, 31 October 2000 (2000-10-31), page II.247, XP009121025 ISSN: 0009-7322
- GEORGE J. ET AL: 'Inhibition of intimal thickening in the rat carotid artery injury model by a nontoxic Ras inhibitor.' ARTERIOSCLER.THROMB.VASC.BIOL. vol. 24, no. 2, February 2004, pages 363 - 368, XP002989159
- MUTO Y. ET AL: 'Prevention of second primary tumors by an acyclic retinoid, polyprenoic acid, in patients with hepatocellular carcinoma.' N.ENGL.J.MED. vol. 334, no. 24, 13 June 1996, pages 1561 - 1567, XP000884177
- MANABE I. ET AL: 'Kekkan Saibo no Signal Seigyo 6 Saibonai Signal o Hyoteki ni Shita Kekkan heikatsukin Keishitsu Henkan Seigyo.' KEKKAN IGAKU. vol. 5, no. 2, May 2004, pages 159 - 166, XP002991809
- KURABAYASHI M. ET AL: 'Domyaku koka 21 Seiki eno Tenbo, Kekkan Heikatsukin Saibo Kino to Keishitsu Henkan.' PHARMA MEDICA. vol. 18, no. 5, May 2005, pages 35 - 41, XP002991810

## Description

### Technical Field

The present invention relates to a medicament comprising an acyclic polyprenyl compound as an active ingredient and having an inhibitory action against the activation of transcription factor KLF5.

### Background Art

In recent years, the incidence of vascular diseases including arteriosclerosis has been rapidly increasing, and development of medicaments for prophylactic or therapeutic treatment of vascular diseases is a very important issue in Japan with coming aging society. Growth of vascular smooth muscle cells is deeply involved in occurrence of restenosis after the vascular reconstructive surgery for coronary arteries, which is performed as a treatment of arteriosclerosis and ischemic heart diseases. However, characteristics of smooth muscles of injured blood vessels and characteristics and properties of blood vessels in the process of ontogeny still remain unclear. Elucidation of the molecular mechanism responsible for differentiation and development of the smooth muscles may possibly trigger development of agents for prophylactic/therapeutic treatment of vascular diseases involving vascular smooth muscles, of which typical example is arteriosclerosis. Further, smooth muscle cells are characterized by plasticity of their trait. While differentiated cells of the constriction type are observed in normal blood vessels, conversion of their trait to that of dedifferentiated smooth muscle cells having proliferation potency is observed in vascular lesions. Understanding the mechanism of dedifferentiation of smooth muscle cells may become a clue of elucidation of pathological conditions of vascular diseases, and further, development of novel therapies.

During studies on expression control of the smooth muscle myosin heavy chain gene, which is specifically expressed in dedifferentiated smooth muscle cells, the inventors of the present invention successfully isolated transcription regulation factor KLF5 (Kruppel-like factors 5)BTEB2/IKLF (henceforth also abbreviated as "KLF5" in the specification), which is responsible for expression regulation of that gene (Circulation Research, 85, pp.182-191, 1999). KLF5 is expressed in the blood vessels during the fetal and neonatal periods, and the expression level thereof decreases in adults. However, expression thereof is induced in blood vessel lesions such as those of arteriosclerosis. Since growth of the smooth muscles is specifically inhibited in an antisense experiment in which the expression of KLF5 is inhibited, KLF5 is considered to be an important factor responsible for growth of the smooth muscles. Therefore, it is expected that if a medicament having an inhibitory action against the activity of KLF5 is developed, the medicament would be useful as a novel medicament for prophylactic and therapeutic treatments of vascular diseases.

The inventors of the present invention found a method for evaluating a substance regulating trait conversion of smooth muscle cells by utilizing the characteristic of KLF5 that it binds to a promoter of a protein which aggravates arteriosclerosis to induce high expression activity thereof, and a method for providing a host cell used for such an evaluation method (Japanese Patent Unexamined Publication (Kokai) No. 11-318450). Further, the inventors of the present invention also successfully constructed a KLF5 knockout mouse (Nature Medicine, 8, pp.856-863, 2002). Embryonic lethality was observed in homozygotes of this knockout mouse, and although heterozygous live births thereof apparently looked normal, decrease in the intimal hyperplasia reaction in response to vascular injury, decrease in cardiac hypertrophy and fibrosis induced by angiotensin II, and the like were found to be remarkable. Further, the platelet derived growth factor (PDGF) level in the heterozygotes was lowered to about half the level in wild type animals, and thus PDGF was found to be a target gene of transcription regulation by KLF5. Further, a retinoic acid derivative (retinoic acid agonist, Am80) inhibited transcription of PDGF-A induced by KLF5 in a reporter assay using cells, and it also inhibited the neointimal formation in injured blood vessels when administered to wild type mice. In contrast, a retinoic acid antagonist promoted transcription of PDGF-A, and it also promoted the neointimal formation in injured blood vessels when administered to wild type mice (Nature Medicine, 8, pp.856-863, 2002).

(2E,4E,6E,10E)-3,7,11,15-Tetramethyl-2,4,6,10,14-hexadecapentanoic acid (hereinafter, this substance is also referred to as "NIK-333" in the specification), one of acyclic polyprenyl compounds, is known to exhibit affinity to retinoic acid binding proteins and retinoic acid receptors, and have a differentiation inducing action and an apoptosis inducing action on hepatocellular carcinoma. Clinically, NIK-333 significantly inhibited recurrence of hepatoma after radical treatment by a long-term administration over one year, and thus the substance has been suggested to have an inhibitory action against hepatoma recurrence. Further, NIK-333 does not substantially induce liver function failure and adverse side effects observed with other retinoids, and therefore the substance is useful as a highly safe medicament (N. Eng. J. Med., 334, pp.1561-1567, 1996). However, it has not been known so far that acyclic polyprenyl compounds inhibit the activation of the transcription factor KLF5, and further, it has not been known that these substances have inhibitory actions against vascular remodeling and arteriosclerosis.

### Disclosure of the Invention

### Object to be Achieved by the Invention

An object of the present invention is to provide a medicament having an inhibitory action against the activation of the transcription control factor KLF5. Further, another object of the present invention is to provide a medicament having an inhibitory action against the activation of the transcription control factor KLF5 and an inhibitory action against vascular remodeling and arteriosclerosis.

### Means for Achieving the Object

In order to achieve the aforementioned objects, the inventors of the present invention made various searches for compounds having an inhibitory action against the activation of the transcription control factor KLF5. As a result, they found that acyclic polyprenyl compounds such as NIK-333 inhibited the activation of the transcription control factor KLF5, and the acyclic polyprenyl compounds had an inhibitory action against vascular remodeling and arteriosclerosis. The present invention was accomplished on the basis of the aforementioned findings.

The present invention thus provides a medicament having an inhibitory action against the activation of the transcription factor KLF5, which comprises an acyclic polyprenyl compound as an active ingredient. The present invention also provides a medicament having an inhibitory action against vascular remodeling, which comprises an acyclic polyprenyl compound as an active ingredient, and a medicament having an inhibitory action against arteriosclerosis, which comprises an acyclic polyprenyl compound as an active ingredient.

According to preferred embodiments, the present invention provides the aforementioned medicament, wherein the acyclic polyprenyl compound is a polyprenylcarboxylic acid; the aforementioned medicament, wherein the acyclic polyprenyl compound is 3,7,11,15-tetramethyl-2,4,6,10,14-hexadecapentanoic acid; and the aforementioned medicament, wherein the acyclic polyprenyl compound is (2E,4E,6E,10E)-3,7,11,15-tetramethyl-2,4,6,10,14-hexadecapentanoic acid. According to more preferred embodiments, the present invention provides the aforementioned medicament, which is in the form of a pharmaceutical composition comprising a pharmaceutically acceptable additive for formulations together with the acyclic polyprenyl compound as an active ingredient; and the aforementioned medicament, which is in the form of a pharmaceutical composition for oral administration.

From another aspect, the present invention also provides use of an acyclic polyprenyl compound for manufacture of the aforementioned medicament; a method for inhibiting the activation of the transcription factor KLF5 in a mammal including human, which comprises the step of administering an effective amount of an acyclic polyprenyl compound to the mammal including human; a method for inhibiting vascular remodeling, which comprises the step of administering an effective amount of an acyclic polyprenyl compound to a mammal including human; and a method for preventing arteriosclerosis, which comprises the step of administering an effective amount of an acyclic polyprenyl compound to a mammal including human.

### Effect of the Invention

The medicament of the present invention has an inhibitory action against the activation of the transcription factor KLF5 and is useful for inhibition of vascular remodeling, arteriosclerosis and the like.

### Brief Description of the Drawings

Fig. 1 shows results of a luciferase assay performed for COS1 cells cotransfected with KLF5 and RARα and loaded with NIK-333.
Fig. 2 shows results of a luciferase assay performed for COS1 cells cotransfected with KLF5 and RARα and loaded with NIK-333 and ATRA.
Fig. 3 shows results of a luciferase assay performed for COS1 cells cotransfected with KLF5, RAR β and RARγ and loaded with NIK-333 and ATRA
Fig. 4 shows results of living cell count of 3T3 cells performed 24, 36, and 48 hours after loading of NIK-333 or ATRA.
Fig. 5 shows results of living cell count of 3T3-KLF5 cells and control cells performed after loading of NIK-333 or ATRA.

### Best Mode for Carrying out the Invention

The acyclic polyprenyl compound used as an active ingredient of the medicament of the present invention means a compound containing several straight chain isoprene units. Type of the functional group at the end of the acyclic polyprenyl compound is not particularly limited. Examples include polyprenyl alcohols (polyprenols) having a primary allylhydroxyl group at the end, compounds consisting of a polyprenol of which end hydroxyl group forms an ester with an organic acid, polyprenylcarboxylic acids having carboxyl group at the end and the like, but not limited to these examples. Polyprenylcarboxylic acids can be preferably used. As the acyclic polyprenyl compound, arbitrary geometrical isomers in pure forms, or arbitrary mixtures of geometrical isomers can be used. The acyclic polyprenyl compounds preferably used for the present invention are 3,7,11,15-tetramethyl-2,4,6,10,14-hexadecapentanoic acids, and (2E,4E,6E,10E)-3,7,11,15-tetramethyl-2,4,6,10,14-hexadecapentanoic acid can be most preferably used. This compound is a known substance described in Japanese Patent Publication (Kokoku) No. 63-32058 and J. Chem. Soc. (c), 2154, 1966, and can be easily prepared by the methods described in the aforementioned publications. Further, other acyclic polyprenyl compounds can also be easily prepared by those skilled in the art by referring to the methods described in the aforementioned publications.

Although acyclic polyprenyl compounds per se may be administered as the medicament of the present invention, it is usually desirable to prepare a pharmaceutical composition together with one or more types of additives for formulations and administer the composition. The administration route of the medicament of the present invention is not particularly limited, and either oral administration or parenteral administration may be chosen. Examples of pharmaceutical compositions suitable for oral administration include tablets (uncoated or sugar-coated tablets and the like), granules, subtilized granules, powders, syrups, solutions, capsules (hard or soft capsules and the like), suspensions, and the like. Examples of preparations suitable for parenteral administration include injections, drip infusions, inhalations, sprays, suppositories, percutaneous absorption agents, transmucosal absorption agents, and the like.

Examples of the additives for formulations include, for example, stabilizers, surfactants, plasticizers, lubricants, solubilizers, buffers, sweeteners, bases, adsorbents, corrigents, binders, suspending agents, brightening agents, coating agents, flavoring agents/aromatizing agents, moistening agent, moisture controlling agents, fillers, antifoaming agents, masticatories, refrigerants, coloring agents, sugar-coating agents, isotonic agents, pH modifiers, softening agents, emulsifiers, tackifiers, adhesion enhancing agents, thickeners, thickening agents, foaming agents, excipients, dispersing agents, propellants, disintegrating agents, disintegrating aids, fragrances, desiccants, antiseptics, preservatives, soothing agents, solvents, dissolving agents, dissolving aids, fluidizing agents and the like, and two or more kinds of these additives can be used in combination. Since specific examples of these additives for formulations are explained in, for example, Pharmaceutical Excipients Directory (ed. by Japan Pharmaceutical Excipients Council, Yakuji Nippo, Ltd.), those skilled in the art can select suitable additives for formulations depending on the form of the pharmaceutical composition and prepare a pharmaceutical composition in a desired form according to methods commonly used in this field. For example, cellulose derivatives, gelatin, plant oils, polyethylene glycol, biologically compatible solvents, and the like can be used as additives for formulations. Further, the specification of PCT/JP03/10440 discloses a soft capsule containing 3,7,11,15-tetramethyl-2,4,6,10,14-hexadecapentanoic acid, and this soft capsule can be suitably used as the medicament of the present invention.

The medicament of the present invention has an inhibitory action against the activation of the transcription factor KLF5 in a mammal including human. The aforementioned action of the medicament of the present invention can be easily confirmed by those skilled in the art according to the methods specifically explained in the examples of the present specification. Further, the medicament of the present invention has an inhibitory action against vascular remodeling and/or arteriosclerosis based on the aforementioned inhibitory action against the activation of the transcription factor KLF5. It is known that, in pathologic conditions of hypertension related to progression of arteriosclerosis and the like, the cellular architecture of blood vessel walls changes in response to various loads on blood vessels, and vascular remodeling occurs. Vascular remodeling means changes in the vascular structure resulting from hemodynamic changes such as changes in blood flow rate and tension of blood vessel wall. The inhibitory action of the medicament of the present invention on vascular remodeling and/or arteriosclerosis can also be easily confirmed by those skilled in the art according to the methods specifically described in the examples of the present specification (for example, evaluation using an inhibitory action against the fibroblast cell growth and cuff-induced injury model and the like) or the like.

A dose and number of administration of the medicament of the present invention are not particularly limited, and the daily dose can be usually selected in the range of about 0.001 to 1200 mg in terms of weight of the active ingredient, and the daily dose for adults is typically about 100 to 1000 mg. The aforementioned doses are desirably adjusted appropriately depending on conditions of patients such as age and body weight, type of disease, and the like. Further, the aforementioned daily dose can be divided into several portions and administered several times a day. Examples

The present invention will be explained more specifically with reference to examples. However, the scope of the present invention is not limited to the following examples.

### Example 1-1: Effect on activation of transcription factor KLF5

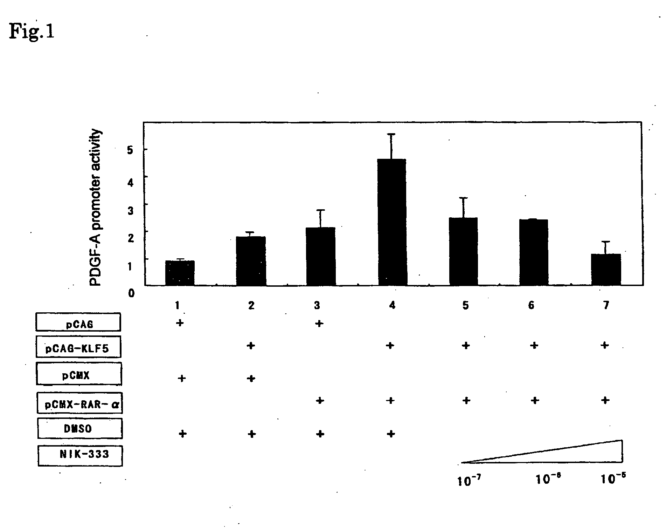

COS1 cells were seeded on a 24-well plate at a ratio of 5 x 10⁴ cells/well and cultured at 37°C in a 5% CO₂ incubator for 12 hours. Transfection was performed by using TransFast (registered trade name, Promega) transfection reagent according to the protocol attached to the reagent. Specifically, the cells were added with pCAG/KLF5 or empty vector thereof (pCAG), pCMX/RARα or empty vector thereof (pCMX) and PDGF-A promoter (-900)-Luciferase and cultured at 37°C in a 5% CO₂ incubator for 48 hours. NIK-333 was dissolved in dimethyl sulfoxide (DMSO), and added at 10⁻⁷ to 10⁻⁵ M. Then, a luciferase assay was performed by using Dual Luciferase Reporter Assay System (Promega). The results are shown in Fig. 1. In contrast to the transfection of the empty vector for KLF5 and the empty vector for RAR α (Lane 1), the transfection of the KLF5 gene alone (Lane 2) and transfection of the RAR a gene alone (Lane 3), the transfection of the KLF5 and RAR α genes (Lane 4) activated the PDGF-A promoter. This KLF5-dependent PDGF-A promoter activation action under overexpression of RAR α was inhibited by NIK-333 (Lanes 5 to 7). Further, the action of NIK-333 showed concentration dependency from 10⁻⁷ M. Accordingly, NIK-333 was found to have an inhibitory action against the activation of the transcription factor KLF5.

### Example 1-2: Effect on activation of transcription factor KLF5

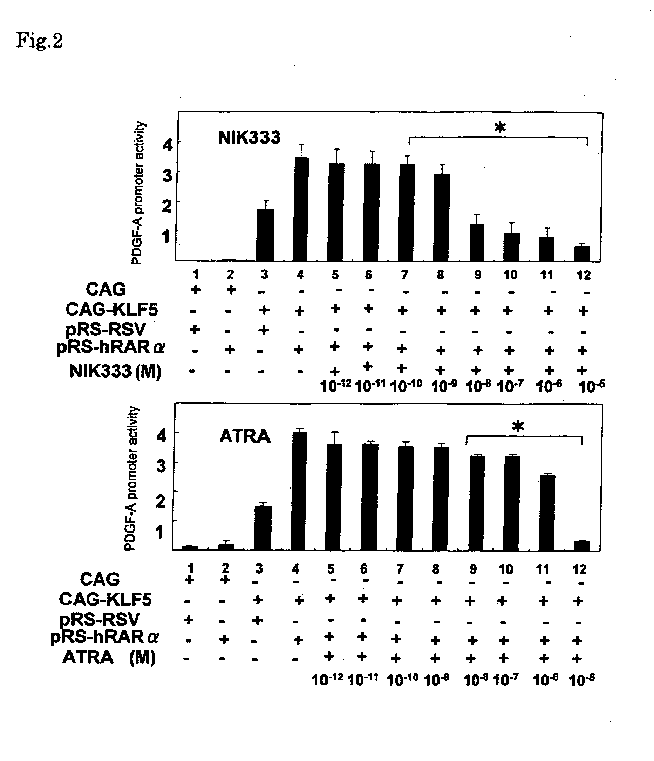

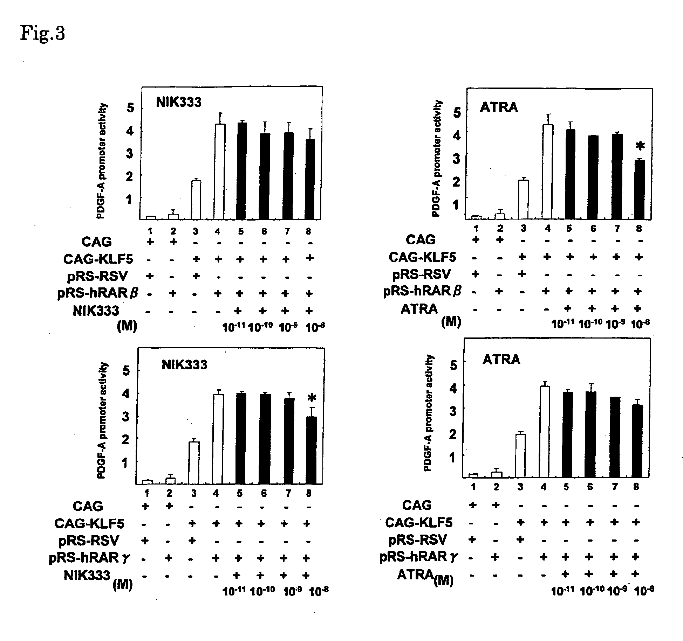

COS1 cells were seeded on a 24-well plate at a rate of 5 x 10⁴ cells/well and cultured overnight at 37°C in a 5% CO₂ incubator. Transfection was performed by using TransFast (registered trade name, Promega) transfection reagent according to the protocol attached to the reagent. Specifically, the cells were added with pCAG/KLF5 or empty vector thereof (pCAG), pRSh/RAR α, pRSh/RAR β, pRSh/RAR γ or empty vector thereof (pRS-RSV) and PDGF-A promoter (-900)-Luciferase and cultured at 37°C in a 5% CO₂ incubator for 48 hours. NIK-333 was dissolved in dimethyl sulfoxide (DMSO), and added at 10⁻¹² to 10⁻⁵ M. Then, a luciferase assay was performed by using Dual Luciferase Reporter Assay System (Promega). The results are shown in Figs. 2 and 3. In contrast to the transfection of the empty vector for KLF5 and the empty vector for RAR α (Lane 1), the transfection of the RAR α gene alone (Lane 2) and transfection of the KLF5 gene alone (Lane 3), the transfection of KLF5 and RARα genes (Lane 4) showed activation of PDGF-A promoter. This KLF5-dependent PDGF-A promoter activation under overexpression of RAR α was inhibited by NIK-333 (Lanes 5 to 12). This inhibitory action was significantly in concentration-dependent manner from 10⁻¹⁰ M and found to be more potent than that of all-trans-retinoic acid (ATRA). Similarly, KLF5-dependent activation of the PDGF-A promoter under overexpression of RAR γ was inhibited by NIK-333, and this inhibitory action was found to be more potent than that of ATRA. On the other hand, it was noted that the KLF5-dependent PDGF-A promoter activation action under overexpression of RAR β was not affected by NIK-333. It was revealed that NIK-333 inhibited the PDGF-A promoter activation action of KLF5 under overexpression of RAR(α or γ), and that this inhibitory action was more potent than that of ATRA. Therefore, NIK-333 was found to have an inhibitory action against the activation of the transcription factor KLF5.

### Example 2: Effect on cell growth of fibroblast 3T3

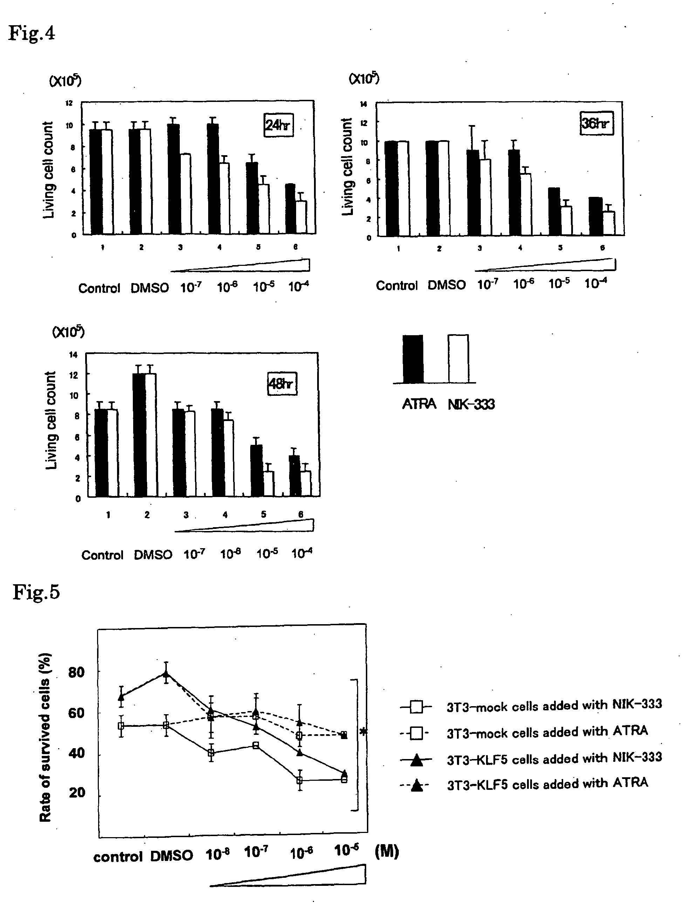

Fibroblast-derived 3T3 cells were seeded on a 24-well plate at a rate of 1 x 10⁶ cells/well and left for 12 hours. Then, NIK-333 and ATRA were added at 10⁻⁷ to 10⁻⁴ M, and living cells were counted 24, 36 and 48 hours later. NIK-333 and ATRA were dissolved in DMSO. As a result, NIK-333 inhibited cell growth in a concentration dependent manner from 10⁻⁷ M, and it was confirmed that this action was more potent than that of ATRA (Fig. 4). Therefore, it is considered that NIK-333 inhibits the fibroblast cell growth, which is thought to be a cause of arteriosclerosis, and hence has an inhibitory action against arteriosclerosis.

### Example 3-1: Effect on mouse cuff-induced injury model

Vascular remodeling analysis was performed by using a cuff-induced injury model. Usefulness of this model in relation to vascular remodeling has been reported (Physiol. Genomics, 2, pp.13-30, 2000; Circulation, 104, pp.2716-2721, 2001; Circulation, 106, pp.847-853, 2002). For this experiment, 8-week old male C57BL6/N mice (10 animals/group) were used. In an amount of 50 mg/kg of NIK-333 dissolved in soybean oil was orally given once a day and every day from 7 days before placement of a cuff (placement of polyethylene tube around blood vessel) to the day before sacrifice. The control group received soybean oil alone in the same manner. After the 7-day pretreatment, the right femoral artery was covered with PE50 (polyethylene tube 50) as a cuff. The animals were sacrificed after 5 weeks. Blood was collected, and the cuff was removed under perfusion fixation via puncture of the left ventricle. For pathological evaluation, hematoxylin/eosin (HE) staining was performed. As a result, the ratio of I/M (intima/media) in vascular intimal hyperplasia resulted from vascular injury due to the cuff decreased in the NIK-333 treatment group compared with the control group.

**[Table 1]**

| Treatment | I/M ratio |
|---|---|
| Solvent (soybean oil) | 0.93 ± 0.35 |
| NIK-333 50 mg/kg | 0.87 ± 0.29 |
| NIK-333 100 mg/kg | 0.75 ± 0.31 |

### Example 3-2: Effect on mouse cuff-induced injury model

Vascular remodeling analysis was performed by using a cuff-induced injury model. Usefulness of this model in relation to vascular remodeling has been reported (Physiol. Genomics, 2, pp.13-30, 2000; Circulation, 104, pp.2716-2721; Circulation, 106, pp.847-853, 2002). For this experiment, 8-week old male C57BL6/N mice (10 animals/group) were used. In an amount of 100 or 200 mg/kg of NIK-333 or 5 or 10 mg/kg of ATRA suspended in soybean oil was orally given once daily every day from 7 days before placement of a cuff (placement of polyethylene tube around blood vessel) to the day before sacrifice. The control group were received soybean oil alone in the same manner. After the 7-day pretreatment, the right femoral artery was covered with PE50 (polyethylene tube 50, outer diameter: 0.965 mm, length: about 1.5 mm) as a cuff. The animals were sacrificed after 5 weeks. Blood was collected, and the cuff was removed under perfusion fixation via puncture of the left ventricle. For pathological evaluation, Elastica van Gieson (EVG) staining was performed. As a result, the ratio of I/M (intima/media) in vascular intimal hyperplasia resulted from vascular injury due to the cuff decreased in the NIK-333 treatment groups compared with the control group. On the other hand, no difference was observed between the ATRA treatment groups and the control group.

**[Table 2]**

| Treatment | I/M ratio (mean ± S.D.) | |
|---|---|---|
| Solvent (soybean oil) | 0.62 ± 0.2 | |
| NIK-333 100 mg/kg | 0.38 ±0.2 | P < 0.05 |
| NIK-333 200 mg/kg | 0.09 ±0.1 | P < 0.01 |
| ATRA 5 mg/kg | 0.64 ± 0.3 | No significant difference |
| ATRA 10 mg/kg | 0.63 ± 0.2 | No significant difference |

### Example 4: Influence on mouse testes weight

Eight-week old male C57BL6/N mice (10 animals/group) were used. In an amount of 50 mg/kg or 100 mg/kg of NIK-333 dissolved in soybean oil was orally given once daily every day for 5 weeks. The control group received soybean oil alone in the same manner. Body weights were measured after 5 weeks. The animals were sacrificed, then the testes were observed by visual observation, and the testes weights were measured. As a result, no difference was observed in body weight and testes weight in the 5-week NIK-333 treatment groups (50 or 100 mg/kg) compared with the control group. Further, no abnormal finding was observed in the testes by the visual observation.

**Table 3**

| Treatment | Body weight (g) | Weight of both testes (g) |
|---|---|---|
| Solvent | 21.1 ± 0.3 | 0.1437 ± 0.0036 |
| NIK-333 50 mg/kg | 20.8 ± 0.3 | 0.1465 ± 0.0043 |
| NIK-333 100 mg/kg | 21.2 ± 0.3 | 0.1360 ± 0.0026 |

### Example 5: Influence on growth of cells with stable KLF5

"3T3-KLF5 cells", cells in which KLF5 was stably expressed (obtained by subcloning cDNA of KLF5 in a 3xFLAG expression vector to prepare a construct of 3xFLAG-KLF5, then transfecting 3T3 cells with the construct, and culturing the transfectants in the presence of G418 for 2 weeks to clone G418 resistant cells, control cells not containing KLF5 which were prepared in the same manner are referred to as 3T3-mock cells), were seeded in DMEM medium containing 10% FBS and penicillin G/streptomycin at a rate of 1 x 10⁵ cells/well (6-well plate) and cultured overnight at 37°C in a 5% CO₂ incubator, and then the medium was changed to DMEM medium containing penicillin G/streptomycin (serum-free). The cells were added with NIK-333 dissolved in DMSO after 2 hours, cultured for 24 hours, and then counted. The results are shown in Fig. 5. When NIK-333 and ATRA dissolved in DMSO were added to the 3T3-mock cells and the 3T3-KLF5 cells at a concentration of 10⁻⁸ to 10⁻⁵M, decrease of the living cell count was observed in a concentration-dependent manner for both types of the cells due to the addition of NIK-333 and ATRA. The action of NIK-333 on the 3T3-mock cells and the 3T3-KLF5 cells was more potent than that of ATRA. After the addition of NIK-333, the survival rate of the 3T3-KLF5 cells was higher than that of the 3T3-mock cells. The cell survival rate differed depending on the presence or absence of KLF5 in the presence of NIK-333, and therefore, it was suggested that the action of NIK-333 was related to the action of KLF5.

### Industrial Applicability

The medicament of the present invention has an inhibitory action against the activation of the transcription factor KLF5, and is useful in inhibition of vascular remodeling, arteriosclerosis and the like.

## Claims

1. A medicament for use in the prophylactic and/or therapeutic treatment of vascular diseases by having an inhibitory action against activation of a transcription factor KLF5, which comprises an acyclic polyprenyl as an active ingredient.

2. The medicament according to claim 1 having an inhibitory action against vascular remodelling.

3. The medicament according to claim 1 having an inhibitory action against arteriosclerosis.

4. The medicament according to any one of claims 1 to 3, wherein the acyclic polyprenyl compound is a polyprenylcarboxylic acid.

5. The medicament according to any one of claims 1 to 3, wherein the acyclic polyprenyl compound is 3,7,11,15-tetramethyl-2,4,6,10,14-hexadecapentanoic acid.

6. The medicament according to any one of claims 1 to 3, wherein the acyclic polyprenyl compound is (2E,4E,6E,10E)-3,7,11,15-tetramethyl-2,4,6,10,14-hexadecapentanoic acid.

7. The medicament according to any one of claims 1 to 6, which is in the form of a pharmaceutical composition containing a pharmaceutically acceptable additive for formulations together with an acyclic polyprenyl compound as an active ingredient.

8. The medicament according to any one of claims 1 to 7, which is in the form of a pharmaceutical composition for oral administration.

## Patentansprüche

1. Arzneimittel zur Verwendung bei der prophylaktischen und/oder therapeutischen Behandlung von Gefäßerkrankungen durch das Vorhandensein einer hemmenden Wirkung gegen die Aktivierung eines Transkriptionsfaktors KLF5, welches ein acyclisches Polyprenyl als einen aktiven Bestandteil enthält.

2. Arzneimittel gemäß Anspruch 1, das eine hemmende Wirkung gegen strukturellen Gefäßwandumbau aufweist.

3. Arzneimittel gemäß Anspruch 1, das eine hemmende Wirkung gegen Arteriosklerose aufweist.

4. Arzneimittel gemäß einem der Ansprüche 1 bis 3, wobei die acyclische Polyprenylverbindung eine Polyprenylcarbonsäure ist.

5. Arzneimittel gemäß einem der Ansprüche 1 bis 3, wobei die acyclische Polyprenylverbindung 3,7,11,15-Tetramethyl-2,4,6,10,14-hexadecapentansäure ist.

6. Arzneimittel gemäß einem der Ansprüche 1 bis 3, wobei die acyclische Polyprenylverbindung (2E,4E,6E,10E)-3,7,11,15-Tetramethyl-2,4,6,10,14-hexadecapentansäure ist.

7. Arzneimittel gemäß einem der Ansprüche 1 bis 6, welches in Form einer pharmazeutischen Zusammensetzung vorliegt, die ein pharmazeutisch akzeptables Additiv für Formulierungen zusammen mit einer acyclischen Polyprenylverbindung als einen aktiven Bestandteil enthält.

8. Arzneimittel gemäß einem der Ansprüche 1 bis 7, welches in Form einer pharmazeutischen Zusammensetzung zur oralen Verabreichung vorliegt.

## Revendications

1. Médicament pour une utilisation dans le traitement prophylactique et/ou thérapeutique de maladies vasculaires, en possédant une action inhibitrice contre l'activation d'un facteur de transcription KLF5, qui comprend un polyprényle acyclique en tant que principe actif.

2. Médicament selon la revendication 1, possédant une action inhibitrice contre le remodelage vasculaire.

3. Médicament selon la revendication 1, possédant une action inhibitrice contre l'artériosclérose.

4. Médicament selon l'une quelconque des revendications 1 à 3, dans lequel le composé de polyprényle acyclique est un acide polyprényl-carboxylique.

5. Médicament selon l'une quelconque des revendications 1 à 3, dans lequel le composé de polyprényle acyclique est l'acide 3,7,11,15-tétraméthyl-2,4,6,10,14-hexadécapentanoïque.

6. Médicament selon l'une quelconque des revendications 1 à 3, dans lequel le composé de polyprényle acyclique est l'acide (2E,4E,6E,10E)-3,7,11,15-tétraméthyl-2,4,6,10,14-hexadécapentanoïque.

7. Médicament selon l'une quelconque des revendications 1 à 6, qui est sous la forme d'une composition pharmaceutique contenant un additif pharmaceutiquement acceptable pour des formulations conjointement avec un composé de polyprényle acyclique en tant que principe actif.

8. Médicament selon l'une quelconque des revendications 1 à 7, qui est sous la forme d'une composition pharmaceutique pour une administration orale.
